# EUROPEAN PATENT APPLICATION

(11) **EP 4 059 504 A1**
(43) Date of publication of application: **21.09.2022**
(21) Application number: 21741344.2
(22) Date of filing: 14.01.2021
(51) Int. Cl.: A61K 31/506, A61P 25/20, A61P 43/00

(54) **DRUG SUBSTANCE OF LEMBOREXANT AND MEDICINAL COMPOSITION COMPRISING SAME**

(30) Priority: 16.01.2020 JP 2020005214
(71) Applicant: Eisai R&D Management Co., Ltd., Tokyo 112-8088 (JP)
(72) Inventor: ABE, Taichi, Kamisu-shi, Ibaraki 314-0255 (JP); AYATA, Yusuke, Kamisu-shi, Ibaraki 314-0255 (JP); SUZUKI, Nobuya, Kakamigahara-shi, Gifu 501-6024 (JP); AKIMOTO, Yurie, Kakamigahara-shi, Gifu 501-6024 (JP); SHIKATA, Futoshi, Kakamigahara-shi, Gifu 501-6024 (JP); ZAIMA, Yasuhiro, Kakamigahara-shi, Gifu 501-6024 (JP); YOSHIDA, Nobuo, Tokyo 112-8088 (JP)
(74) Representative: Hoffmann Eitle
(86) International application number: PCT/JP2021/001027
(87) International publication number: WO 2021/145373

(57) **Abstract**

There is provided a pharmaceutical composition comprising lemborexant exhibiting a favorable dissolution profile by using a granular material having a 90% cumulative diameter (D90) of 64 µm or less as a lemborexant active pharmaceutical ingredient.

## Description

### Technical Field

The present invention relates to a lemborexant active pharmaceutical ingredient and a pharmaceutical composition comprising the same.

### Background Art

Orexin receptors are G protein-coupled receptors that are dominantly present in the brain. The endogenous ligands of the orexin receptors, orexin-A and orexin-B, are expressed by neurons that are localized in the hypothalamic. Orexin-Ais a peptide of 33 amino acids, and orexin-B is composed of 28 amino acids (Non-Patent Literature 1). There are two subtypes of orexin receptors, OX1 and OX2, and OX1 preferentially binds to orexin-A, while OX2 binds to both orexin-A and orexin-B. It has been suggested that orexins stimulate food consumption in rats and orexin signaling can play a role in principal feedback mechanisms for adjusting eating behaviors (Non-Patent Literature 1). Orexins have also been observed to control the arousal-sleep state (Non-Patent Literature 2). Orexins are also capable of playing a role in brain changes accompanied by opioid and nicotine addiction (Non-Patent Literatures 3 and 4) and ethanol addition (Non-Patent Literature 5). Furthermore, it has been suggested that orexins play a role in some stress reactions (Non-Patent Literature 6).

It is known that compounds such as (1R,2S)-2-(((2,4-dimethylpyrimidine-5-yl)oxy)methyl)-2-(3-fluorophenyl)-N-(5-fluoropyridin-2-yl)cyclopropanecarboxamide (also called "lemborexant") are strong orexin receptor antagonists, and these compounds are useful for treatments for sleep disorders such as insomnia and other therapeutic applications (Patent Literatures 1 and 2). Results of a dissolution test of tablets containing 1 mg to 25 mg of lemborexant have been reported (Patent Literature 3).

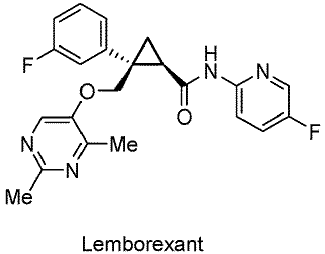

### Citation List

### Patent Literature

[Patent Literature 1] WO 2012/039371
[Patent Literature 2] WO 2013/123240
[Patent Literature 3] WO 2016/063995

### Non-Patent Literature

[Non-Patent Literature 1] Sakurai T. et al., Cell, 1998, Vol. 92, pp. 573 to 585
[Non-Patent Literature 2] Chemelli R. M. et al., Cell, 1999, Vol. 98, pp. 437 to 451
[Non-Patent Literature 3] S. L. Borgland et al., Neuron, 2006, Vol. 49, pp 598 to 601
[Non-Patent Literature 4] C. J. Winrow et al., Neuropharmacology, 2010, Vol. 58, pp. 185 to 194
[Non-Patent Literature 5] J. R. Shoblock et al., Psychopharmacology, 2011, Vol. 215, pp. 191 to 203
[Non-Patent Literature 6] T. Ida et al., Biochem. Biophys. Res. Commun., 2000, Vol. 270, pp. 318 to 323

### Summary of Invention

### Technical Problem

It is desirable that a pharmaceutical composition has favorable physicochemical properties. An object of the present invention is to provide a pharmaceutical composition comprising lemborexant, which exhibits a favorable dissolution profile.

### Solution to Problem

The inventors conducted extensive studies in order to solve the above problems, and as a result, found that a pharmaceutical composition comprising lemborexant exhibits a favorable dissolution profile by using a lemborexant granular material having a certain 90% cumulative diameter (D90) as an active pharmaceutical ingredient.

Specifically, the present invention relates to the following [1] to [15].
[1] A lemborexant active pharmaceutical ingredient, which is a granular material having a 90% cumulative diameter (D90) of 64 µm or less.
[2] A pharmaceutical composition comprising lemborexant and a pharmaceutically acceptable additive, produced by using a lemborexant active pharmaceutical ingredient, which is a granular material having a 90% cumulative diameter (D90) of 64 µm or less.
[3] The pharmaceutical composition according to [2], wherein a dissolution rate of lemborexant is 50% or more within 15 minutes from the start time of a dissolution test in a dissolution medium (2nd fluid for dissolution test (pH 6.8) in Japanese Pharmacopoeia 16th Edition, 900 mL, 37±0.5°C) using a paddle apparatus (rotation speed: 50 rpm) in accordance with the paddle method.
[4] The pharmaceutical composition according to [2], wherein a dissolution rate of lemborexant is 50% or more within 15 minutes from the start time of a dissolution test in a dissolution medium (water, 900 mL, 37±0.5°C) using a paddle apparatus (rotation speed: 50 rpm) in accordance with the paddle method.
[5] The pharmaceutical composition according to [2], wherein a dissolution rate of lemborexant is 50% or more within 15 minutes from the start time of a dissolution test in a dissolution medium (2nd fluid for dissolution test (pH 6.8) in Japanese Pharmacopoeia 16th Edition, 900 mL, 37±0.5°C) in Japanese Pharmacopoeia 16th Edition, 900 mL, 37±0.5°C) using a paddle apparatus (rotation speed: 100 rpm) in accordance with the paddle method.
[6] The pharmaceutical composition according to [2], wherein a dissolution rate of lemborexant is 80% or more within 15 minutes from the start time of a dissolution test in a dissolution medium (0.1 mol/L hydrochloric acid, 900 mL, 37±0.5°C) using a paddle apparatus (rotation speed: 50 rpm) in accordance with the paddle method.
[7] The pharmaceutical composition according to any one of [2] to [6], wherein the pharmaceutical composition is a tablet.
[8] The pharmaceutical composition according to any one of [2] to [7], which comprises 2.5 mg to 10 mg of lemborexant.
[9] The pharmaceutical composition according to [8], which comprises 10 mg of lemborexant.
[10] The pharmaceutical composition according to [8], which comprises 5 mg of lemborexant.
[11] The pharmaceutical composition according to [8], which comprises 2.5 mg of lemborexant.
[12] A pharmaceutical composition comprising lemborexant and a pharmaceutically acceptable additive, wherein a dissolution rate of lemborexant is 80% or more within 15 minutes from the start time of a dissolution test in a dissolution medium (0.1 mol/L hydrochloric acid, 900 mL, 37±0.5°C) and a dissolution rate of lemborexant is 50% or more within 15 minutes from the start time of a dissolution test in another dissolution medium (2nd fluid for dissolution test (pH 6.8) in Japanese Pharmacopoeia 16th Edition, 900 mL, 37±0.5°C) using a paddle apparatus (rotation speed: 50 rpm) in accordance with the paddle method.
[13] The pharmaceutical composition according to [12], wherein a lemborexant active pharmaceutical ingredient is a granular material having a 90% cumulative diameter (D90) of 64 µm or less.
[14] The pharmaceutical composition according to [12], produced by using a lemborexant active pharmaceutical ingredient, which is a granular material having a 90% cumulative diameter (D90) of 64 µm or less.
[15] A method of producing a pharmaceutical composition comprising lemborexant and a pharmaceutically acceptable additive, comprising a step of mixing a lemborexant active pharmaceutical ingredient, which is a granular material having a 90% cumulative diameter (D90) of 64 µm or less and a pharmaceutically acceptable additive.

### Advantageous Effects of Invention

The present invention can provide a pharmaceutical composition comprising lemborexant exhibiting a favorable dissolution profile by using a granular material having a 90% cumulative diameter (D90) of 64 µm or less as a lemborexant active pharmaceutical ingredient.

### Brief Description of Drawings

Fig. 1(a) and 1(b) show the results of a dissolution test of tablets containing 2.5 mg and 10 mg of lemborexant in 0.1 mol/L hydrochloric acid shown in Table 6 and Table 7.
Fig. 2(a) and 2(b) show the results of a dissolution test of tablets containing 2.5 mg of lemborexant in the dissolution media shown in Table 8 and Table 9.
Fig. 3(a) and 3(b) show the results of a dissolution test of tablets containing 2.5 mg of lemborexant in the dissolution media shown in Table 10 and Table 12.
Fig. 4 shows the results of a dissolution test of tablets containing 2.5 mg of lemborexant in water shown in Table 11.
Fig. 5(a) and 5(b) show the results of a dissolution test of tablets containing 10 mg of lemborexant in the dissolution media shown in Table 13 and Table 14.
Fig. 6(a) and 6(b) show the results of a dissolution test of tablets containing 10 mg of lemborexant in the dissolution media shown in Table 15 and Table 17.
Fig. 7 shows the results of a dissolution test of tablets containing 10 mg of lemborexant in water shown in Table 16.
Fig. 8(a), 8(b) and 8(c) show the results of a dissolution test of tablets containing 2.5 mg, 10 mg and 5 mg of lemborexant in the dissolution media shown in Table 21, Table 22 and Table 23.

### Description of Embodiments

Hereinafter, the meanings of signs, terminologies and the like used in the present specification will be described, and the present specification will be described in detail.

In one embodiment of the present invention, lemborexant is a free base.

Lemborexant can be produced in accordance with a known method, for example, the method described in WO 2012/039371 and WO 2013/123240.

Since the particle size of the lemborexant active pharmaceutical ingredient affects a dissolution profile in some dissolution media, a granular material having a 90% cumulative diameter (D90) of 64 µm or less in a particle cumulative distribution (volume distribution) is preferable.

In one embodiment of the present invention, the lemborexant active pharmaceutical ingredient is a granular material having a 90% cumulative diameter (D90) of 64 µm or less.

Since the lemborexant active pharmaceutical ingredient produced in accordance with a desired method may be a granular material having a 90% cumulative diameter (D90) larger than 64 µm, the lemborexant active pharmaceutical ingredient can be pulverized in accordance with a known method.

The pulverization method is not particularly limited, and for example, a plate beater (100UPZ, 160UPZ), a pin mill (100UPZ, 160UPZ) or the like can be used.

In one embodiment of the present invention, when the lemborexant active pharmaceutical ingredient produced in accordance with a desired method is pulverized, as shown in Table 1, the lemborexant active pharmaceutical ingredient may be a granular material having a 90% cumulative diameter (D90) of 64 µm or less.

**[Table 1]**

| Lot | Particle size (D90 (µm)) |
|---|---|
| 1 | 47 |
| 2 | 18 |
| 3 | 48 |
| 4 | 32 |
| 5 | 39 |
| 6 | 23 |
| 7 | 40 |
| 8 | 35 |
| 9 | 34 |
| Average | 35.1 |
| Standard deviation | 10.0 |
| Average + upper confidence limit^{a} | 65 |

| | |
|---|---|
| ^{a}upper confidence limit=3 times standard deviation of batch analysis data | |

The particle size of the granular material, which is a lemborexant active pharmaceutical ingredient, can be measured by a laser diffraction/scattering method.

The particle size can be measured under the following conditions.

A sample suspension is prepared by dispersing about 30 mg of a lemborexant active pharmaceutical ingredient in 50 mL of a 0.1% polysorbate 20 aqueous solution as a dispersion solvent. The sample suspension is put into a diffractometer and an appropriate amount of dispersion solvent is added. The particle size distribution of the sample suspension is measured, and the value of the particle size of the sample is recorded as a cumulative diameter distribution.

Particle size distribution measurement conditions for the diffractometer may be as follows.
Flow rate: 50%
Number of measurements: 3 times
Measurement time: 30 seconds
Shape: Non-spherical
Transparency: Transparent
Particle refractive index: 1.81
Solvent refractive index: 1.33

In one embodiment of the present invention, the pharmaceutical composition is a granular material comprising lemborexant and one or more pharmaceutically acceptable additives, and in which the lemborexant active pharmaceutical ingredient has a 90% cumulative diameter (D90) of 64 µm or less. A pharmaceutical composition can be produced by mixing pharmaceutically acceptable additives with a lemborexant active pharmaceutical ingredient, which is a granular material having a 90% cumulative diameter (D90) of 64 µm or less. The pharmaceutical composition according to the present invention can be produced in accordance with a known method, for example, the method described in the general rules for preparation of Japanese Pharmacopoeia 16th Edition.

In one embodiment of the present invention, the additives include excipients, disintegrants, lubricants, surfactants, sweeteners, fragrance materials, stabilizers, plasticizers and the like, but the present invention is not limited thereto.

In one embodiment of the present invention, the pharmaceutical composition is a solid preparation, and in one embodiment, the pharmaceutical composition is an oral solid preparation.

In one embodiment of the present invention, the pharmaceutical composition is a tablet.

In one embodiment of the present invention, the pharmaceutical composition comprises 2.5 mg to 10 mg of lemborexant. The content of lemborexant in the pharmaceutical composition may be 2.5 mg, 5 mg or 10 mg.

In this specification, regarding the dissolution test, the test can be performed in accordance with the paddle method described in Japanese Pharmacopoeia, US Pharmacopoeia, and European Pharmacopoeia (for example, the paddle method specified in the 6.10 dissolution test method in Japanese Pharmacopoeia 16th Edition) under the following conditions.
Apparatus: Paddle apparatus
Volume of dissolution medium: 900 mL
Temperature of dissolution medium: 37±0.5°C
Dissolution medium: 0.1 mol/L hydrochloric acid, the 1st fluid for dissolution test (pH 1.2) in Japanese Pharmacopoeia 16th Edition, diluted McIlvaine buffer solution (pH 5.0), 2nd fluid for dissolution test (pH 6.8) in Japanese Pharmacopoeia 16th Edition or water
Paddle rotation speed: 50 rpm or 100 rpm

The 1st fluid for dissolution test is a solution prepared by dissolving 2.0 g of sodium chloride in 7.0 mL of hydrochloric acid and water to make 1000mL.

The 2nd fluid for dissolution test is a solution prepared by adding 1 volume of water to 1 volume of a phosphate buffer solution at a pH of 6.8.

In one embodiment of the present invention, the pharmaceutical composition comprises lemborexant and one or more pharmaceutically acceptable additives, and is produced by using a lemborexant active pharmaceutical ingredient, which is a granular material having a 90% cumulative diameter (D90) of 64 µm or less, and for such a pharmaceutical composition, a dissolution rate of lemborexant is 50% or more within 15 minutes from the start time of a dissolution test in a dissolution medium (2nd fluid for dissolution test (pH 6.8) in Japanese Pharmacopoeia 16th Edition, 900 mL, 37±0.5°C) using a paddle apparatus (rotation speed: 50 rpm) in accordance with the paddle method.

In one embodiment of the present invention, the pharmaceutical composition comprises lemborexant and one or more pharmaceutically acceptable additives, and is produced by using a lemborexant active pharmaceutical ingredient, which is a granular material having a 90% cumulative diameter (D90) of 64 µm or less, and for such a pharmaceutical composition, a dissolution rate of lemborexant is 50% or more within 15 minutes from the start time of a dissolution test in a dissolution medium (water, 900 mL, 37±0.5°C) using a paddle apparatus (rotation speed: 50 rpm) in accordance with the paddle method.

In one embodiment of the present invention, the pharmaceutical composition comprises lemborexant and one or more pharmaceutically acceptable additives, and is produced by using a lemborexant active pharmaceutical ingredient, which is a granular material having a 90% cumulative diameter (D90) of 64 µm or less, and for such a pharmaceutical composition, a dissolution rate of lemborexant is 50% or more within 15 minutes from the start time of a dissolution test in a dissolution medium (2nd fluid for dissolution test (pH 6.8) in Japanese Pharmacopoeia 16th Edition, 900 mL, 37±0.5°C) in Japanese Pharmacopoeia 16th Edition, 900 mL, 37±0.5°C) using a paddle apparatus (rotation speed: 100 rpm) in accordance with the paddle method.

In one embodiment of the present invention, the pharmaceutical composition comprises lemborexant and one or more pharmaceutically acceptable additives, and is produced by using a lemborexant active pharmaceutical ingredient, which is a granular material having a 90% cumulative diameter (D90) of 64 µm or less, and for such a pharmaceutical composition, a dissolution rate of lemborexant is 80% or more within 15 minutes from the start time of a dissolution test in a dissolution medium (0.1 mol/L hydrochloric acid, 900 mL, 37±0.5°C) using a paddle apparatus (rotation speed: 50 rpm) in accordance with the paddle method.

In one embodiment of the present invention, the pharmaceutical composition comprises lemborexant and one or more pharmaceutically acceptable additives, and a dissolution rate of lemborexant is 80% or more within 15 minutes from the start time of a dissolution test in a dissolution medium (0.1 mol/L hydrochloric acid, 900 mL, 37±0.5°C) and a dissolution rate of lemborexant is 50% or more within 15 minutes from the start time of a dissolution test in another dissolution medium (2nd fluid for dissolution test (pH 6.8) in Japanese Pharmacopoeia 16th Edition, 900 mL, 37±0.5°C) using a paddle apparatus (rotation speed: 50 rpm) in accordance with the paddle method.

In one embodiment of the present invention, the pharmaceutical composition can be appropriately administered to patients in accordance with its dosage form.

In one embodiment of the present invention, the dose of lemborexant varies depending on the degree of symptoms, an age, a sex, a body weight, a dosage form/type of salt, a specific type of disease, and the like, but generally, for adults, a daily oral dose is 2.5 mg to 10 mg, or is selected from among 2.5 mg, 5 mg, and 10 mg.

### Examples

Hereinafter, the present invention will be described in detail using examples. However, the present invention is not limited to these examples. In addition, abbreviations that are used in the following examples are common abbreviations well known to those skilled in the art, and several abbreviations are as described below.

Chemical shifts in a proton nuclear magnetic resonance (¹H-NMR) spectrum are recorded in δ units (ppm) with respect to tetramethylsilane, and coupling constants are recorded in Hertz (Hz). In patterns, s, d, br and m means singlet, doublet, broad and multiplet, respectively.

¹H-NMR spectra and ¹³C-NMR spectra were measured using Varian400, a 500 MHz spectrometer, or a JNM-AL400 type nuclear magnetic resonance apparatus (400 MHz) (commercially available from JEOL Ltd.).

"Room temperature" in the following examples generally indicate about 10°C to about 35°C. Unless particularly otherwise described, % indicates weight percentages.

The lemborexant of the present invention was produced by the following method. The compound of the formula A can be produced by, for example, the production method described in WO 2012/039371 or WO 2013/123240.

### Production of (1R,2S)-2-(((2,4-dimethylpyrimidine-5yl)oxy)methyl)-2-(3-fluorophenyl)-N-(5-fluoropyridin-2-yl)cyclopropanecarboxamide (lemborexant)

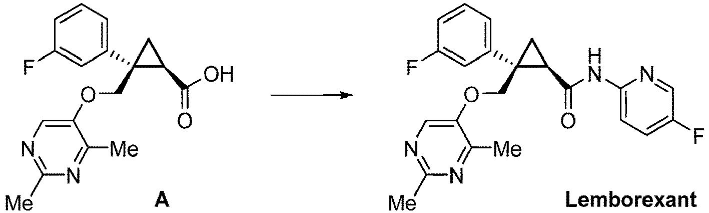

A mixture of (1R,2S)-2-{[(2,4-dimethylpyrimidine-5-yl)oxy]methyl}-2-(3-fluorophenyl)cyclopropane carboxylic acid (62.4 kg, 197 mol, 1.00 equiv.), 5-fluoropyridine-2-amine (24.3 kg, 217 mol, 1.10 equiv.), ethyl acetate (499 L), N,N-diisopropylethylamine (53.5 kg, 414 mol, 2.10 equiv.), and 1-propanephosphonic anhydride (50% ethyl acetate solution) (188 kg, 296 mol, 1.50 equiv.) was heated at an external temperature of 70°C, and the end of a reaction was confirmed by HPLC analysis. After the reaction mixture was cooled, 312 L of purified water was added thereto, and the mixture was stirred and then left to stand. After the aqueous layer was discharged, a sodium carbonate aqueous solution (68.9 kg of sodium carbonate and 312 L of purified water) was added to the organic layer, and the mixture was stirred and then left to stand. After the aqueous layer was discharged, purified water (187 L) was added to the organic layer, and the mixture was stirred and then left to stand. The aqueous layer was discharged, purified water (187L) was added to the organic layer, and the mixture was stirred and then left to stand. The aqueous layer was discharged and the organic layer was filtered. A clarifying filtration line was rinsed with ethyl acetate, and the solvent was then partially distilled away under reduced pressure. A mixture was prepared by adding ethyl acetate to a concentrated residue (containing substantially 75.3 kg of lemborexant) such that the total volume reached 256 L, and the mixture was heated and dissolved under stirring at an external temperature of 60°C. n-Heptane (12.8 kg) was added thereto and the mixture was cooled to 45°C or lower. Ethyl acetate (31 L) was added thereto, the mixture was cooled to 35°C or lower, and n-heptane (670 kg) was then added thereto. After that, a suspension was cooled to 10°C or lower, and a solid in the mixture was collected by filtration and washed with a mixture of ethyl acetate and n-heptane. The obtained solid was dried at an external temperature of 60°C or lower under reduced pressure, and thereby lemborexant (70 kg) with a yield of 87% was obtained.

Conditions for HPLC for confirming the above-described reaction:
Reagents and mobile phases:
   Purified water
   Acetonitrile (Kanto Chemical Co., Inc. Catalog No. 01031-2B, for high performance liquid chromatograph, or equivalent product)
   Trifluoroacetic acid (Wako Pure Chemical Industries, Ltd., Catalog No. 208-02741 reagent special grade, or equivalent product)
   Mobile phase A: Mixture of water and trifluoroacetic acid (1000/1, v/v)
   Mobile phase B: Mixture of acetonitrile and trifluoroacetic acid (1000/1, v/v)
   Solution: Mixture of water, acetonitrile, and trifluoroacetic acid (500/500/1, v/v/v)
   HPLC apparatus needle rinse agent: Mixture of water and acetonitrile (100/900, v/v)
   Solution: Mixture of water and acetonitrile (1/1, v/v)
   Standard sample of (1R,2S)-2-{[(2,4-dimethylpyrimidine-5-yl)oxy]methyl}-2-(3-fluorophenyl)cyclopropane carboxylic acid)
HPLC conditions
Detector: Shimadzu SPD-20A ultraviolet absorptiometer (measurement wavelength: 283 nm) or equivalent product
Column: Stainless steel pipe having an inner diameter of 4.6 mm and a length of 15 cm and filled with 3.5 µm octadecylsilylated silica gel for liquid chromatography

Example: SunFire C18 (Waters) (equivalent to USP packing L1) Column temperature: Constant temperature around 40°C
Mobile phases:
A: Mixture of water and trifluoroacetic acid (1000/1, v/v)
B: Mixture of acetonitrile and trifluoroacetic acid (1000/1, v/v) Flow rate: Constant flow rate of about 1.0 mL/min

Gradient conditions:
Time (minutes) Percentage of mobile phase B (%)

| | |
|---|---|
| 0 | 5>Linear gradient |
| 7 | 30 |
| 20 | 30>Linear gradient |
| 34 | 100 |
| 39 | 100 |
| 39.01 | 5 |
| 50 | Stop |

Injection volume: 5 µL
Sample rack temperature: Constant temperature around 10°C
Needle rinse agent: Mixture of water and acetonitrile (10/90, v/v)
Area measurement target range: Up to 34 minutes

### NMR data

(1R,2S)-2-(((2,4-dimethylpyrimidine-5-yl)oxy)methyl)-2-(3-fluorophenyl)-N-(5-fluoropyridin-2-yl)cyclopropanecarboxamide:
¹H NMR (500 MHz, DMSO-d₆) δ 11.19 (s, 1H), 8.31 (d, J = 3.0 Hz, 1H), 8.12 (s, 1H), 7.94-7.85 (m, 1H), 7.62 (tt, J = 8.7, 3.1 Hz, 1H), 7.44 (dd, J = 10.6, 1.5 Hz, 1H), 7.41-7.40 (m, 1H), 7.39 (s, 1H), 7.14-7.06 (m, 1H), 4.67 (d, J = 10.2 Hz, 1H), 4.29 (t, J = 9.9 Hz, 1H), 2.63 (t, J = 7.0 Hz, 1H), 2.38 (s, 3H), 2.03 (s, 3H), 1.76-1.64 (m, 1H), 1.49 (dd, J = 8.0, 4.8 Hz, 1H);
¹³C NMR (125 MHz, DMSO-d₆) δ168.68, 161.98 (d, JCF= 242.3 Hz), 158.46,155.15, 155.38 (d, JCF = 247.9 Hz), 148.90, 148.51, 145.00 (d, JCF = 7.7 Hz), 139.37, 135.15 (d, JCF= 24.9 Hz), 130.06 (d, JCF = 8.4 Hz), 125.05 (d, JCF = 19.5Hz), 124.70 (d, JCF = 2.6 Hz), 115.71 (d, JCF = 21.7 Hz), 114.20 (d, JCF = 4.1 Hz), 113.70 (d, JCF = 20.9 Hz), 70.80, 34.09 (d, JCF = 1.9 Hz), 26.90,24.38, 18.37, 17.78.

### Example 1: Preparation of lemborexant active pharmaceutical ingredient having various particle sizes

A number of lemborexant active pharmaceutical ingredients having different particle sizes were prepared using an unpulverized product of a lemborexant active pharmaceutical ingredient by changing a rotation speed of 160UPZ (plate beater, pin mill).

One kg of the unpulverized lemborexant active pharmaceutical ingredient was weighed out in advance in a container, and put into a pulverizer (160UPZ plate beater, opening 1 mm mesh screen) while measuring the time with a stopwatch. The injection was manually performed with a shovel. The rotation speed was 4,000, 5,600, 8,000, 9,500 rpm, and pulverization was performed under conditions of a feed rate of 10 kg/h (lots S1, S2, S3, S4, and S6). However, the lot S6 was prepared by pulverizing once under conditions of a rotation speed of 5,600 rpm and a feed rate of 60 kg/h (lot S5), then pulverizing with a 160UPZ pin mill at 5,600 rpm, and then continuously pulverizing at 10,000 rpm. Table 2 shows the pulverization conditions and the obtained 90% cumulative diameter (D90) after pulverization together.

The 90% cumulative diameter (D90) was measured with a particle size analyzer (Microtrack MT3300EXII). A sample suspension was prepared by dispersing about 30 mg of a lemborexant active pharmaceutical ingredient in 50 mL of a dispersion solvent 0.1% polysorbate 20 aqueous solution. The sample suspension was put into a particle size analyzer, and an appropriate amount of a dispersion solvent was added thereto. The particle size distribution of the sample suspension was measured, and the value of the particle size of the sample was recorded as a cumulative diameter distribution.

The measurement conditions for the particle size analyzer are as follows.
Flow rate: 50%
Number of measurements: 3 times
Measurement time: 30 seconds
Shape: Non-spherical
Transparency: Transparent
Particle refractive index: 1.81
Solvent refractive index: 1.33

**[Table 2]**

| Lot | Rotation speed (rpm) | Feed rate (kg/h) | D90 (µm) |
|---|---|---|---|
| S1 | 4000 | 10 | 98 |
| S2 | 5600 | 10 | 79 |
| S3 | 8000 | 10 | 64 |
| S4 | 9500 | 10 | 56 |
| S5 | 5600 | 60 | 110 |
| S6 | 10000 | 10 | 20 |

### Example 2: Manufacturing of tablets containing 2.5 mg and 10 mg of lemborexant

Tablets containing 2.5 mg and 10 mg of lemborexant were manufactured using each lot (S1, S2, S3, S4, and S6) obtained in Example 1.

Table 3 shows components and compositions of the manufactured tablets.

**[Table 3]**

| Component, composition and batch quantity of tablets containing 2.5 mg and 10 mg of lemborexant | | | | |
|---|---|---|---|---|
| Component | Amount | | | |
| | 2.5 mg | | 10 mg | |
| | mg/tablet | g/batch | mg/tablet | g/batch |
| Internal phase | | | | |
| Lemborexant | 2.5 | 27.08 | 10.0 | 108.3 |
| Lactose hydrate | 96.38 | 1044.12 | 88.88 | 962.87 |
| Low-substituted hydroxypropyl cellulose | 10.8 | 117.0 | 10.8 | 117.0 |
| Hydroxypropyl cellulose | 3.6 | 39.0 | 3.6 | 39.0 |
| Purified water | q.s. | 163 | q.s. | 163 |

| External phase | | | | |
|---|---|---|---|---|
| Low-substituted hydroxypropyl cellulose | 6.0 | 65.0 | 6.0 | 65.0 |
| Magnesium stearate | 0.72 | 7.8 | 0.72 | 7.8 |
| Total weight | 120.0 | 1300.0 | 120.0 | 1300.0 |

Tablets containing 2.5 mg and 10 mg of lemborexant were manufactured by mixing, granulation, drying, milling, blending and tableting processes. Lemborexant active pharmaceutical ingredient, lactose hydrate, and low-substituted hydroxypropylcellulose were charged into a wet high-shear granulator, and mixed for 5 minutes. A solution obtained by dissolving hydroxypropyl cellulose in purified water was added into the wet high-shear granulator containing the obtained mixture and the mixture was granulated for 3 minutes. The obtained wet granules were dried using a fluid bed dryer (inlet air temperature of 70°C) until the outlet air temperature was higher than 38°C. The obtained dried granules were milled using a milling machine with a screen diameter of 1.0 mm. Low-substituted hydroxypropyl cellulose and magnesium stearate were weighed out according to the yield of granules. The dried granules, low-substituted hydroxypropyl cellulose and magnesium stearate were charged into a tumble type mixer, and mixed for 16 minutes. The obtained mixture was tableted with a tableting machine at a compression force of 1,150 kgf to obtain tablets.

### Example 3: Dissolution test

### (1) Apparatuses and materials

The apparatuses and materials used in the dissolution test are listed in Table 4.

**[Table 4]**

| Apparatuses and materials | |
|---|---|
| Item | apparatuses and materials |
| Apparatus | Dissolution system (apparatus 2): Evolution 6100 (DISTEK) |
| | HPLC system: LC-2010C_{HT} (Shimadzu Corporation) |
| | Chemical balance |
| Material | Acetonitrile (HPLC grade) |
| | 70% perchloric acid (analytical grade) |
| | 0.1 mol/L hydrochloric acid |
| | 1st fluid for dissolution test (Japanese Pharmacopoeia 16th Edition) |
| | Diluted McIlvaine buffer solution (pH 5.0) listed in Japanese Guidelines for Bioequivalence Studies |
| | 2nd fluid for dissolution test (Japanese Pharmacopoeia 16th Edition) |
| | Purified water |

### (2) Dissolution test conditions

Table 5 shows conditions for the dissolution test performed.

Each test was performed with 6 or 12 tablets manufactured in accordance with the method described in Example 2.

**[Table 5]**

| Conditions for dissolution test | | | | |
|---|---|---|---|---|
| Apparatus | Rotation speed | Dissolution media | Volume of solution | Temperature |
| Apparatus 2 (paddle) | 50 rpm | 0.1 mol/L hydrochloric acid | 900 mL | 37±0.5°C |
| | | pH 1.2 (1st fluid for dissolution test (Japanese Pharmacopoeia 16th Edition)) | | |
| | | pH 5.0 (diluted McIlvaine buffer solution) | | |
| | | pH 6.8 (2nd fluid for dissolution test (Japanese Pharmacopoeia 16th Edition)) | | |
| | | Water | | |
| | 100 rpm | pH 6.8 (2nd fluid for dissolution test (Japanese Pharmacopoeia 16th Edition)) | | |

### (3) Analysis procedure

The dissolution test was performed in accordance with the paddle apparatus listed in Japanese Pharmacopoeia 16th Edition. The tablets were tested using 900 mL of 0.1 mol/L hydrochloric acid, a 1st fluid for dissolution test (pH 1.2), a diluted McIlvaine buffer solution, a 2nd fluid for dissolution test (pH 6.8) or water as a dissolution medium at a paddle rotation speed of 50 rpm or 100 rpm. A portion of the dissolution medium was taken out through a filter at a specified time. The standard solution was prepared to have a concentration of lemborexant corresponding to the normal concentration of the sample solution. The amount of lemborexant released was determined by HPLC or an ultraviolet-visible absorbance measurement method. Conditions for HPLC and conditions for the ultraviolet-visible absorbance measurement method are shown below.

### <Conditions for HPLC>

Detector: Ultraviolet absorptiometer (measurement wavelength: 283 nm)
Column: an inner diameter of 4.6 mm and a length of 7.5 cm (YMC-TriartC18(YMC))
Filler: 3 µm octadecylsilylated silica gel for liquid chromatography
Column temperature: 40°C
Mobile phase: water/acetonitrile/70% perchloric acid (550:450:1, v/v/v)
Flow rate: 1.2 mL/min
Injection volume: 50 µL
Sample rack temperature: 25°C
Analysis time: 5 minutes

### <Conditions for ultraviolet-visible absorbance measurement method (measurement by this method only for 0.1 mol/L hydrochloric acid)>

Measurement wavelength: 233 nm and 400 nm
Control: Dissolution medium

### (4) Results

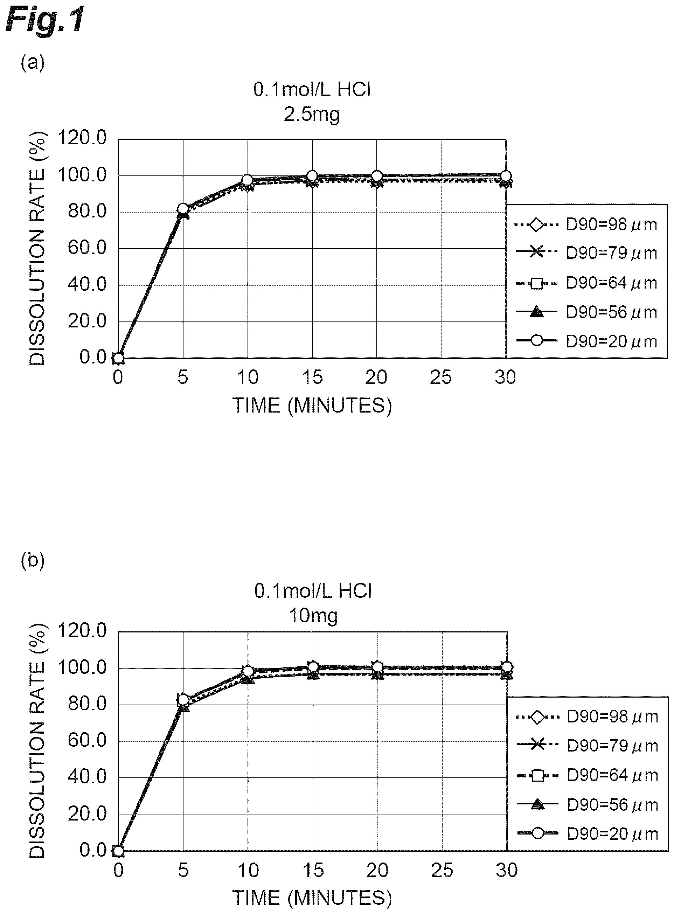

The test was performed with 6 or 12 tablets, and an average value thereof is shown. Table 6 and Table 7 and Fig. 1 show the results of the dissolution test of 2.5 mg and 10 mg tablets of lemborexant when 0.1 mol/L hydrochloric acid was used as the dissolution medium. All tablets were completely released in 0.1 mol/L hydrochloric acid within 15 minutes regardless of the particle size and content of the lemborexant active pharmaceutical ingredient.

**[Table 6]**

| Dissolution profiles of tablets containing 2.5 mg of lemborexant in 0.1 mol/L hydrochloric acid (rotation speed 50 rpm) | | | | | |
|---|---|---|---|---|---|
| Particle size (D90) | Dissolution rate (%) | | | | |
| | 5 min. | 10 min. | 15 min. | 20 min. | 30 min. |
| 98 µm | 81.0 | 95.8 | 96.9 | 97.1 | 97.0 |
| 79 µm | 79.4 | 95.2 | 97.5 | 97.6 | 97.4 |
| 64 µm | 82.4 | 97.6 | 99.8 | 100.0 | 100.7 |
| 56 µm | 82.3 | 96.8 | 98.5 | 98.3 | 98.3 |
| 20 µm | 81.8 | 97.8 | 100.0 | 100.1 | 100.5 |

**[Table 7]**

| Dissolution profiles of tablets containing 10 mg of lemborexant in 0.1 mol/L hydrochloric acid (rotation speed 50 rpm) | | | | | |
|---|---|---|---|---|---|
| Particle | Dissolution rate (%) | | | | |
| size (D90) | 5 min. | 10 min. | 15 min. | 20 min. | 30 min. |
| 98 µm | 80.3 | 95.3 | 96.8 | 96.7 | 96.7 |
| 79 µm | 79.1 | 94.8 | 96.8 | 96.7 | 96.7 |
| 64 µm | 82.7 | 97.6 | 99.9 | 99.8 | 99.8 |
| 56 µm | 78.8 | 94.3 | 97.1 | 97.0 | 96.9 |
| 20 µm | 82.1 | 98.4 | 101.0 | 100.9 | 100.9 |

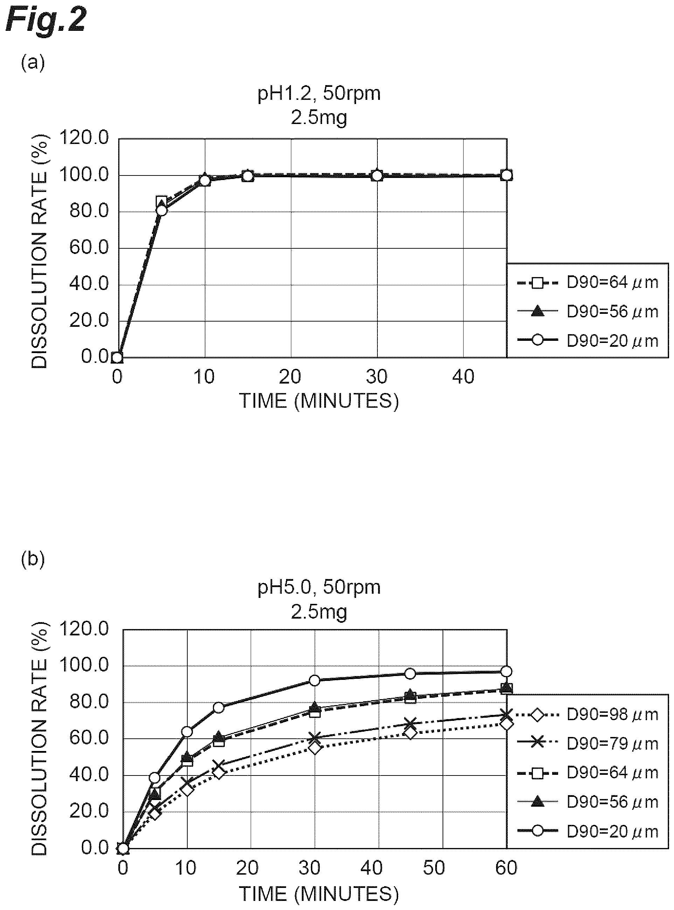

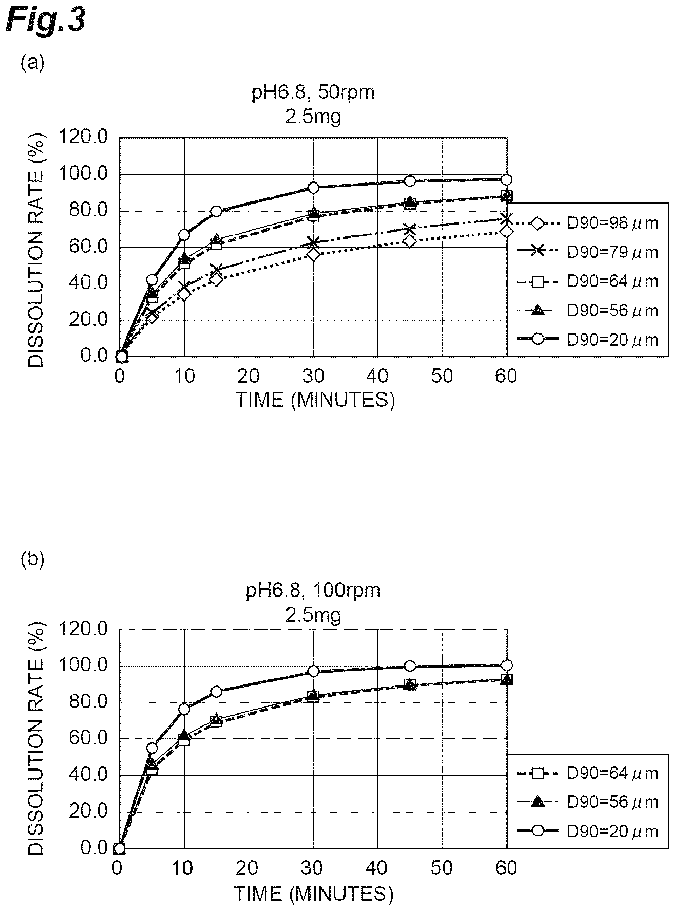

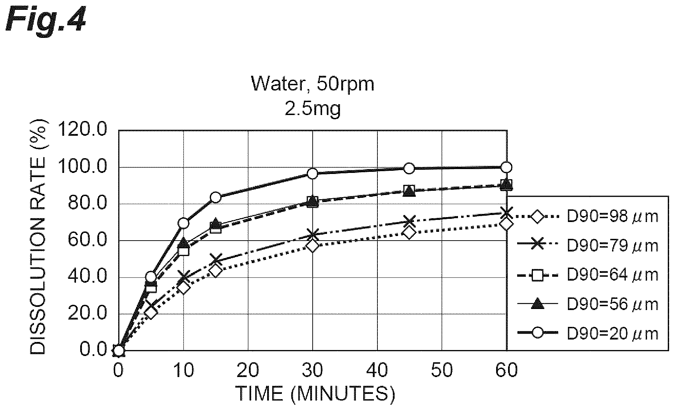

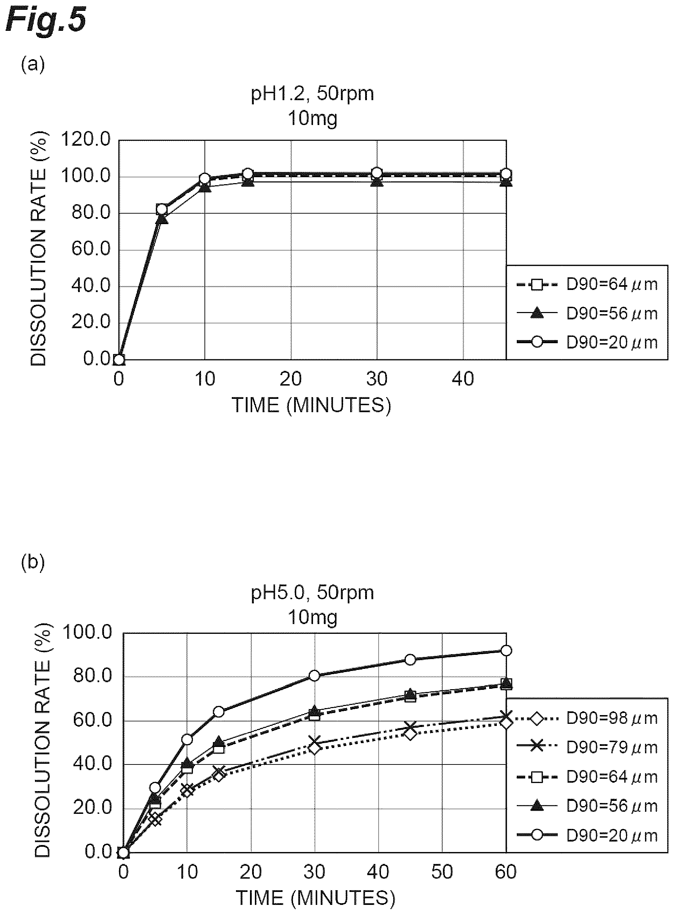

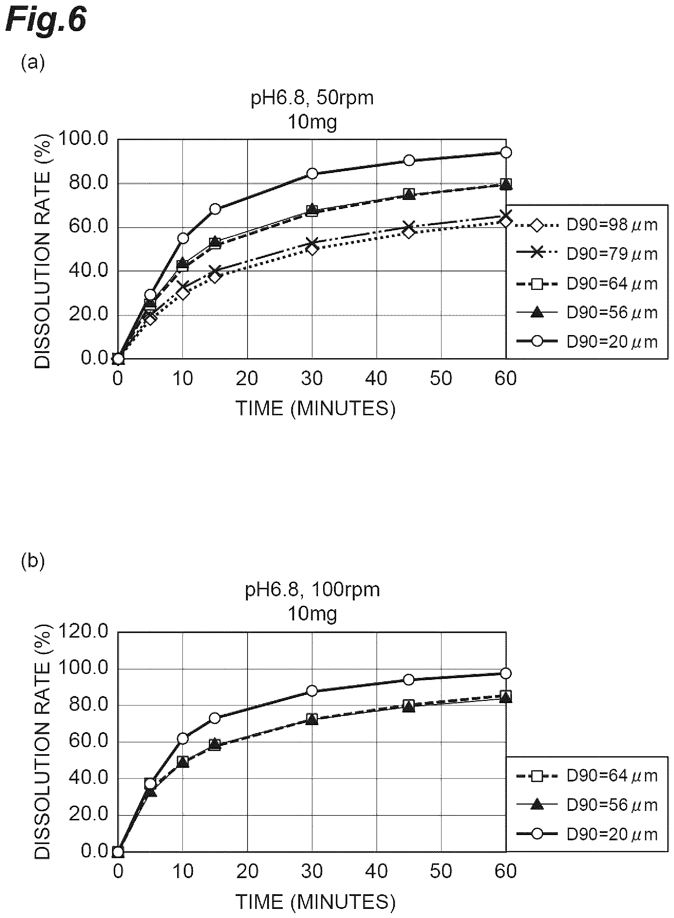

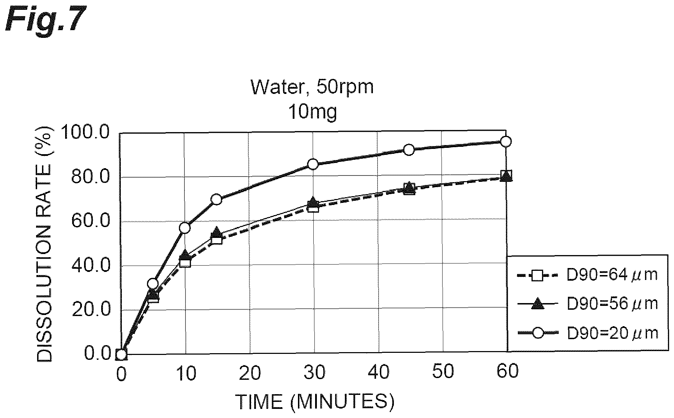

Tables 8 to 12 and Fig. 2 to Fig. 4 show the results of the dissolution test of 2.5 mg tablets of lemborexant in different dissolution media. In addition, Tables 13 to 17 and Fig. 5 to Fig. 7 show the results of the dissolution test of 10 mg tablets of lemborexant in different dissolution media. Here, "N/T" in the following tables indicates Not Tested. Tablets in which the lemborexant active pharmaceutical ingredient had a particle size (D90) of 64 µm or less exhibited a favorable dissolution profile in all dissolution media for 2.5 mg and 10 mg tablets of lemborexant. On the other hand, it was confirmed that, in tablets in which the lemborexant active pharmaceutical ingredient had a particle size (D90) of 79 µm and 98 µm, the dissolution rate (%) decreased in a diluted McIlvaine buffer solution, a 2nd fluid for dissolution test (pH 6.8) and water for 2.5 mg and 10 mg tablets of lemborexant.

Therefore, it was suggested that it was possible to provide a homogeneous pharmaceutical composition exhibiting a favorable dissolution profiles by setting the particle size (D90) of the lemborexant active pharmaceutical ingredient to 64 µm or less.

In addition, the dissolution test of Example 3 is a test method in line with Japanese Pharmacopoeia, US Pharmacopoeia and European Pharmacopoeia as described in the <711> Dissolution section of US Pharmacopoeia (USP42). Based on such a test method, it was confirmed that 2.5 mg and 10 mg tablets exhibited a favorable dissolution profile. Since 2.5 mg and 10 mg tablets were formulations in which the difference in the amount of the active pharmaceutical ingredient in one tablet was replaced with a lactose hydrate, a similar favorable dissolution profile was expected to be exhibited for 2.5 mg to 10 mg tablets according to a bracketing method, for example, 5 mg tablet.

**[Table 8]**

| Dissolution profiles of tablets containing 2.5 mg of lemborexant at pH 1.2 (1st fluid for dissolution test (Japanese Pharmacopoeia)) (rotation speed 50 rpm) | | | | | | |
|---|---|---|---|---|---|---|
| Particle size (D90) | Dissolution rate (%) | | | | | |
| | 5 min. | 10 min. | 15 min. | 30 min. | 45 min. | 60 min. |
| 98 µm | N/T | N/T | N/T | N/T | N/T | N/T |
| 79 µm | N/T | N/T | N/T | N/T | N/T | N/T |
| 64 µm | 85.0 | 98.5 | 100.5 | 100.6 | 100.2 | N/T |
| 56 µm | 83.3 | 98.5 | 100.1 | 100.2 | 100.3 | N/T |
| 20 µm | 80.9 | 97.2 | 99.9 | 99.4 | 99.7 | N/T |

**[Table 9]**

| Dissolution profiles of tablets containing 2.5 mg of lemborexant at pH 5.0 (diluted McIlvaine buffer solution) (rotation speed 50 rpm) | | | | | | |
|---|---|---|---|---|---|---|
| Particle size (D90) | Dissolution rate (%) | | | | | |
| | 5 min. | 10 min. | 15 min. | 30 min. | 45 min. | 60 min. |
| 98 µm | 19.3 | 32.1 | 40.8 | 55.0 | 63.1 | 68.3 |
| 79 µm | 21.7 | 35.9 | 45.6 | 60.6 | 68.4 | 73.3 |
| 64 µm | 31.0 | 48.0 | 59.0 | 75.2 | 82.5 | 86.9 |
| 56 µm | 30.3 | 49.1 | 60.7 | 76.8 | 83.6 | 87.5 |
| 20 µm | 38.8 | 63.9 | 77.4 | 92.1 | 95.8 | 96.8 |

**[Table 10]**

| Dissolution profiles of tablets containing 2.5 mg of lemborexant at pH 6.8 (2nd fluid for dissolution test (Japanese Pharmacopoeia)) (rotation speed 50 rpm) | | | | | | |
|---|---|---|---|---|---|---|
| Particle size (D90) | Dissolution rate (%) | | | | | |
| | 5 min. | 10 min. | 15 min. | 30 min. | 45 min. | 60 min. |
| 98 µm | 22.0 | 34.2 | 42.3 | 55.8 | 63.5 | 68.6 |
| 79 µm | 24.0 | 38.5 | 47.7 | 62.6 | 70.6 | 75.8 |
| 64 µm | 32.9 | 50.8 | 61.6 | 77.1 | 84.1 | 88.1 |
| 56 µm | 34.6 | 53.5 | 64.3 | 78.7 | 84.8 | 88.3 |
| 20 µm | 41.8 | 67.0 | 79.7 | 92.9 | 96.3 | 97.3 |

**[Table 11]**

| Dissolution profiles of tablets containing 2.5 mg of lemborexant in water (rotation speed 50 rpm) | | | | | | |
|---|---|---|---|---|---|---|
| Particle size (D90) | Dissolution rate (%) | | | | | |
| | 5 min. | 10 min. | 15 min. | 30 min. | 45 min. | 60 min. |
| 98 µm | 20.7 | 34.6 | 43.4 | 57.0 | 64.3 | 69.0 |
| 79 µm | 23.9 | 39.2 | 48.7 | 63.2 | 70.6 | 75.3 |
| 64 µm | 34.9 | 54.9 | 66.4 | 81.2 | 87.3 | 90.5 |
| 56 µm | 37.6 | 57.7 | 68.5 | 82.0 | 87.2 | 89.9 |
| 20 µm | 40.2 | 69.5 | 83.7 | 96.6 | 99.5 | 100.1 |

**[Table 12]**

| Dissolution profiles of tablets containing 2.5 mg of lemborexant at pH 6.8 (2nd fluid for dissolution test (Japanese Pharmacopoeia)) (rotation speed 100 rpm) | | | | | | |
|---|---|---|---|---|---|---|
| Particle size (D90) | Dissolution rate (%) | | | | | |
| | 5 min. | 10 min. | 15 min. | 30 min. | 45 min. | 60 min. |
| 98 µm | N/T | N/T | N/T | N/T | N/T | N/T |
| 79 µm | N/T | N/T | N/T | N/T | N/T | N/T |
| 64 µm | 43.4 | 59.5 | 68.9 | 83.0 | 89.3 | 92.8 |
| 56 µm | 45.9 | 61.8 | 70.9 | 84.1 | 89.8 | 92.9 |
| 20 µm | 55.0 | 76.3 | 86.0 | 96.9 | 99.6 | 100.4 |

**[Table 13]**

| Dissolution profiles of tablets containing 10 mg of lemborexant at pH 1.2 (1st fluid for dissolution test (Japanese Pharmacopoeia)) (rotation speed 50 rpm) | | | | | | |
|---|---|---|---|---|---|---|
| Particle size (D90) | Dissolution rate (%) | | | | | |
| | 5 min. | 10 min. | 15 min. | 30 min. | 45 min. | 60 min. |
| 98 µm | N/T | N/T | N/T | N/T | N/T | N/T |
| 79 µm | N/T | N/T | N/T | N/T | N/T | N/T |
| 64 µm | 82.2 | 98.3 | 100.7 | 100.6 | 100.7 | N/T |
| 56 µm | 76.8 | 94.4 | 97.2 | 97.3 | 97.2 | N/T |
| 20 µm | 82.2 | 99.1 | 101.9 | 101.8 | 101.8 | N/T |

**[Table 14]**

| Dissolution profiles of tablets containing 10 mg of lemborexant at pH 5.0 (diluted McIlvaine buffer solution) (rotation speed 50 rpm) | | | | | | |
|---|---|---|---|---|---|---|
| Particle size (D90) | Dissolution rate (%) | | | | | |
| | 5 min. | 10 min. | 15 min. | 30 min. | 45 min. | 60 min. |
| 98 µm | 15.2 | 27.0 | 34.7 | 46.9 | 54.1 | 58.9 |
| 79 µm | 15.3 | 28.0 | 36.5 | 49.5 | 57.1 | 62.1 |
| 64 µm | 22.6 | 37.8 | 47.6 | 62.7 | 70.9 | 76.2 |
| 56 µm | 24.0 | 40.1 | 50.1 | 64.7 | 72.2 | 76.9 |
| 20 µm | 29.0 | 51.4 | 64.1 | 80.4 | 87.8 | 92.0 |

**[Table 15]**

| Dissolution profiles of tablets containing 10 mg of lemborexant at pH 6.8 (2nd fluid for dissolution test (Japanese Pharmacopoeia)) (rotation speed 50 rpm) | | | | | | |
|---|---|---|---|---|---|---|
| Particle size (D90) | Dissolution rate (%) | | | | | |
| | 5 min. | 10 min. | 15 min. | 30 min. | 45 min. | 60 min. |
| 98 µm | 18.2 | 29.9 | 37.4 | 50.0 | 57.4 | 62.7 |
| 79 µm | 20.1 | 32.4 | 40.2 | 53.0 | 60.3 | 65.4 |
| 64 µm | 24.8 | 41.3 | 51.7 | 66.7 | 74.6 | 79.7 |
| 56 µm | 25.6 | 43.2 | 53.4 | 67.7 | 75.0 | 79.4 |
| 20 µm | 29.3 | 54.6 | 68.2 | 84.2 | 90.2 | 94.1 |

**[Table 16]**

| Dissolution profiles of tablets containing 10 mg of lemborexant in water (rotation speed 50 rpm) | | | | | | |
|---|---|---|---|---|---|---|
| Particle size (D90) | Dissolution rate (%) | | | | | |
| | 5 min. | 10 min. | 15 min. | 30 min. | 45 min. | 60 min. |
| 98 µm | N/T | N/T | N/T | N/T | N/T | N/T |
| 79 µm | N/T | N/T | N/T | N/T | N/T | N/T |
| 64 µm | 25.7 | 41.8 | 51.5 | 66.1 | 73.7 | 78.5 |
| 56 µm | 26.8 | 44.1 | 54.0 | 67.7 | 74.5 | 78.6 |
| 20 µm | 31.9 | 57.0 | 69.7 | 85.0 | 91.5 | 94.8 |

**[Table 17]**

| Dissolution profiles of tablets containing 10 mg of lemborexant at pH 6.8 (2nd fluid for dissolution test (Japanese Pharmacopoeia)) (rotation speed 100 rpm) | | | | | | |
|---|---|---|---|---|---|---|
| Particle size (D90) | Dissolution rate (%) | | | | | |
| | 5 min. | 10 min. | 15 min. | 30 min. | 45 min. | 60 min. |
| 98 µm | N/T | N/T | N/T | N/T | N/T | N/T |
| 79 µm | N/T | N/T | N/T | N/T | N/T | N/T |
| 64 µm | 34.4 | 48.8 | 57.8 | 72.4 | 80.3 | 85.5 |
| 56 µm | 32.4 | 49.2 | 58.4 | 72.2 | 79.3 | 83.8 |
| 20 µm | 37.2 | 61.8 | 73.0 | 87.5 | 94.0 | 97.5 |

### Example 4: Manufacturing of tablets containing 2.5 mg, 5 mg and 10 mg of lemborexant

Table 18 shows components and compositions of the manufactured tablets.

**[Table 18]**

| Component, composition, and batch quantity of tablets containing 2.5 mg, 5 mg and 10 mg of lemborexant | | | | | | |
|---|---|---|---|---|---|---|
| Component | Amount | | | | | |
| | 2.5 mg | | 5 mg | | 10 mg | |
| | mg/tablet | kg/batch | mg/tablet | kg/batch | mg/tablet | kg/batch |
| Internal phase | | | | | | |
| Lemborexant | 2.5 | 0.271 | 5 | 0.542 | 10 | 1.083 |
| Lactose hydrate | 96.38 | 10.441 | 93.88 | 10.17 | 88.88 | 9.629 |
| Low-substituted hydroxypropyl cellulose | 10.8 | 1.17 | 10.8 | 1.17 | 10.8 | 1.17 |
| Hydroxypropyl cellulose | 3.6 | 0.39 | 3.6 | 0.39 | 3.6 | 0.39 |
| Purified water | q.s. | 3.68 | q.s. | 2.88 | q.s. | 2.88 |
| External phase | | | | | | |
| Low-substituted hydroxypropyl cellulose | 6 | 0.65 | 6 | 0.65 | 6 | 0.65 |
| Magnesium stearate | 0.72 | 0.078 | 0.72 | 0.078 | 0.72 | 0.078 |
| Total weight | 120.0 | | 120.0 | | 120.0 | |

Tablets containing 2.5 mg, 5 mg and 10 mg of lemborexant were manufactured by mixing, granulation, drying, milling, blending and tableting processes. Lemborexant active pharmaceutical ingredient (32 µm (D90)), a lactose hydrate, and low substituted hydroxypropyl cellulose were charged into a et high-shear granulator, and mixed for 5 minutes. A solution obtained by dissolving hydroxypropyl cellulose in purified water was added into the et high-shear granulator comprising the obtained mixture and the mixture was granulated for 3 minutes. The obtained wet granules were dried in a fluid bed dryer (inlet air temperature of 70°C) until the outlet air temperature was higher than 40°C. The obtained dried granules were milled using a milling machine with a screen diameter of 0.8 mm. Low-substituted hydroxypropyl cellulose and magnesium stearate were weighed out according to the yield of granules. The dried granules, low-substituted hydroxypropyl cellulose and magnesium stearate were charged into a tumble type mixer, and mixed for 14 minutes. The obtained mixture was tableted with a tableting machine at a compression force of 700 kgf, 1,150 kgf and 1,600 kgf to obtain tablets.

### Example 5: Dissolution test

### (1) Apparatuses and materials

The apparatuses and materials used in the dissolution test are listed in Table 19.

**[Table 19]**

| Item | Apparatuses and materials |
|---|---|
| Apparatus | Dissolution system (apparatus 2): Evolution 6100 (DISTEK) |
| | Ultraviolet-visible spectrophotometer: UV-1700 (Shimadzu Corporation) |
| | Chemical balance |
| Material | Acetonitrile (HPLC grade) |
| | 0.1 mol/L hydrochloric acid |

### (2) Dissolution test conditions

Table 20 shows conditions for the dissolution test performed.

Each test was performed with 6 tablets manufactured in accordance with the method described in Example 4.

**[Table 20]**

| Conditions for dissolution test | | | | |
|---|---|---|---|---|
| Apparatus | Rotation speed | Dissolution medium | Volume of solution | Temperature |
| Apparatus 2 (paddle) | 50 rpm | 0.1 mol/L hydrochloric acid | 900 mL | 37±0.5°C |

### (3) Analysis procedure

The dissolution test was performed in accordance with the paddle apparatus listed in Japanese Pharmacopoeia 16th Edition. The tablets were tested using 900 mL of 0.1 mol/L hydrochloric acid as a dissolution medium at a paddle rotation speed of 50 rpm. A portion of the dissolution medium was taken out through a filter at a specified time. The standard solution was prepared to have a concentration of lemborexant corresponding to the normal concentration of the sample solution. The amount of lemborexant released was determined by an ultraviolet-visible absorbance measurement method. Conditions for the ultraviolet-visible absorbance measurement method are shown below.

### <Conditions for ultraviolet-visible absorbance measurement method>

Measurement wavelength: 233 nm and 400 nm
Control: Dissolution medium

### (4) Results

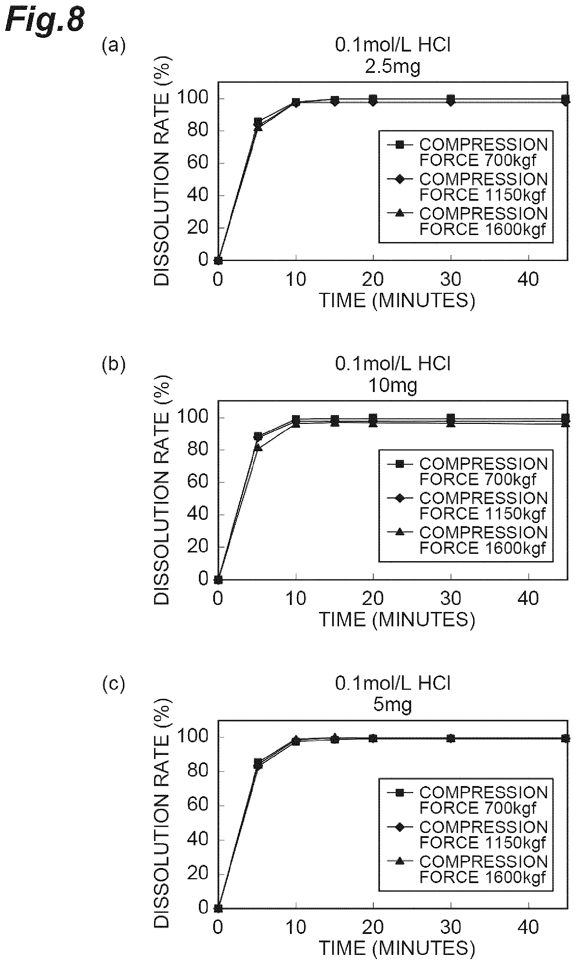

The test was performed with 6 tablets, and an average value thereof is shown. Table 21 to Table 23 and Fig. 8 show the results of the dissolution test of 2.5 mg, 5 mg and 10 mg tablets of lemborexant manufactured at different compression force when 0.1 mol/L hydrochloric acid was used as the dissolution medium.

**[Table 21]**

| Dissolution profiles of tablets containing 2.5 mg of lemborexant manufactured at each compression force in 0.1 mol/L hydrochloric acid (rotation speed 50 rpm) | | | | | | |
|---|---|---|---|---|---|---|
| Compression force (kgf) | Dissolution rate (%) | | | | | |
| | 5 min. | 10 min. | 15 min. | 20 min. | 30 min. | 45 min. |
| 700 | 86.2 | 98.2 | 99.1 | 99.2 | 99.2 | 99.3 |
| 1150 | 83.8 | 96.8 | 97.3 | 97.5 | 97.5 | 97.4 |
| 1600 82.5 | | 97.6 | 99.3 | 99.5 | 99.6 | 99.7 |

**[Table 22]**

| Dissolution profiles of tablets containing 10 mg of lemborexant manufactured at each compression force in 0.1 mol/L hydrochloric acid (rotation speed 50 rpm) | | | | | | |
|---|---|---|---|---|---|---|
| Compression force (kgf) | Dissolution rate (%) | | | | | |
| | 5 min. | 10 min. | 15 min. | 20 min. | 30 min. | 45 min. |
| 700 | 89 | 98.2 | 99.1 | 99.8 | 100.1 | 100.1 |
| 1150 | 88.2 | 97.8 | 98.4 | 98.5 | 98.7 | 98.8 |
| 1600 | 82.2 | 96.6 | 97.4 | 97.5 | 97.6 | 97.6 |

**[Table 23]**

| Dissolution profiles of tablets containing 5 mg of lemborexant manufactured at each compression force in 0.1 mol/L hydrochloric acid (rotation speed 50 rpm) | | | | | | |
|---|---|---|---|---|---|---|
| Compression force (kgf) | Dissolution rate (%) | | | | | |
| | 5 min. | 10 min. | 15 min. | 20 min. | 30 min. | 45 min. |
| 700 | 85.6 | 98.3 | 99.4 | 99.6 | 99.8 | 99.7 |
| 1150 | 82.8 | 97.5 | 99.1 | 99.1 | 99.1 | 99.3 |
| 1600 | 83.9 | 99.1 | 100.3 | 100.4 | 100.5 | 100.5 |

In the dissolution test of 2.5 mg tablets, the 5 mg tablets and the 10 mg tablets using 0.1 mol/L hydrochloric acid, it was confirmed that there was no effect on dissolution in a compression force range of 700 kgf to 1,600 kgf.

## Claims

1. A lemborexant active pharmaceutical ingredient, which is a granular material having a 90% cumulative diameter (D90) of 64 µm or less.

2. A pharmaceutical composition comprising lemborexant and a pharmaceutically acceptable additive, produced by using a lemborexant active pharmaceutical ingredient, which is a granular material having a 90% cumulative diameter (D90) of 64 µm or less.

3. The pharmaceutical composition according to claim 2,
wherein a dissolution rate of lemborexant is 50% or more within 15 minutes from the start time of a dissolution test in a dissolution medium (2nd fluid for dissolution test (pH 6.8) in Japanese Pharmacopoeia 16th Edition, 900 mL, 37±0.5°C) using a paddle apparatus (rotation speed: 50 rpm) in accordance with the paddle method.

4. The pharmaceutical composition according to claim 2,
wherein a dissolution rate of lemborexant is 50% or more within 15 minutes from the start time of a dissolution test in a dissolution medium (water, 900 mL, 37±0.5°C) using a paddle apparatus (rotation speed: 50 rpm) in accordance with the paddle method.

5. The pharmaceutical composition according to claim 2,
wherein a dissolution rate of lemborexant is 50% or more within 15 minutes from the start time of a dissolution test in a dissolution medium (2nd fluid for dissolution test (pH 6.8) in Japanese Pharmacopoeia 16th Edition, 900 mL, 37±0.5°C) in Japanese Pharmacopoeia 16th Edition, 900 mL, 37±0.5°C) using a paddle apparatus (rotation speed: 100 rpm) in accordance with the paddle method.

6. The pharmaceutical composition according to claim 2,
wherein a dissolution rate of lemborexant is 80% or more within 15 minutes from the start time of a dissolution test in a dissolution medium (0.1 mol/L hydrochloric acid, 900 mL, 37±0.5°C) using a paddle apparatus (rotation speed: 50 rpm) in accordance with the paddle method.

7. The pharmaceutical composition according to any one of claims 2 to 6, wherein the pharmaceutical composition is a tablet.

8. The pharmaceutical composition according to any one of claims 2 to 7, which comprises 2.5 mg to 10 mg of lemborexant.

9. The pharmaceutical composition according to claim 8, which comprises 10 mg of lemborexant.

10. The pharmaceutical composition according to claim 8, which comprises 5 mg of lemborexant.

11. The pharmaceutical composition according to claim 8, which comprises 2.5 mg of lemborexant.

12. A pharmaceutical composition comprising lemborexant and a pharmaceutically acceptable additive, wherein a dissolution rate of lemborexant is 80% or more within 15 minutes from the start time of a dissolution test in a dissolution medium (0.1 mol/L hydrochloric acid, 900 mL, 37±0.5°C) and a dissolution rate of lemborexant is 50% or more within 15 minutes from the start time of a dissolution test in another dissolution medium (2nd fluid for dissolution test (pH 6.8) in Japanese Pharmacopoeia 16th Edition, 900 mL, 37±0.5°C) using a paddle apparatus (rotation speed: 50 rpm) in accordance with the paddle method.

13. The pharmaceutical composition according to claim 12,
wherein a lemborexant active pharmaceutical ingredient is a granular material having a 90% cumulative diameter (D90) of 64 µm or less.

14. The pharmaceutical composition according to claim 12, produced by using a lemborexant active pharmaceutical ingredient, which is a granular material having a 90% cumulative diameter (D90) of 64 µm or less.

15. A method of producing a pharmaceutical composition comprising lemborexant and a pharmaceutically acceptable additive, comprising
a step of mixing a lemborexant active pharmaceutical ingredient, which is a granular material having a 90% cumulative diameter (D90) of 64 µm or less and a pharmaceutically acceptable additive.
